# EUROPEAN PATENT APPLICATION

(11) **EP 1 365 031 A1**
(43) Date of publication of application: **26.11.2003**
(21) Application number: 02010274.5
(22) Date of filing: 21.05.2002
(51) Int. Cl.: C12Q 1/68

(54) **Method for detection of somatic mutations using mass spectometry**

(71) Applicant: MTM Laboratories AG, 69120 Heidelberg (DE)
(72) Inventor: Bonk, Thomas, 69118 Heidelberg (DE); von Knebel Doeberitz, Magnus, 69118 Heidelberg (DE); Becker, Cord-Michael, 91088 Bubenreuth (DE)

(57) **Abstract**

The present invention relates to a method for detection of somatic mutations in samples. The method is based on detection of somatic mutations by means of mass spectrometry, whereby a comparison of complex signal patterns comprising aberrant signals compared to wild type and/or integrals over particular peaks within the spectra is used for assessing the presence or absence of mutations in nucleotide sequences. According to the method disclosed herein a ratio of the signal integrals of a reference peak and a peak to be analysed are built. The peaks employed for the comparison are chosen to allow for a reproducible correlation of the ratios of the integrals to the particular somatic mutation phenotype.

## Description

The present invention relates to a method for detection of somatic mutations in samples. The method is based on detection of somatic mutations by means of mass spectrometry, whereby the presence of specific peak patterns within the spectrum is used for assessing the presence or absence of mutations in nucleotide sequences. According to the method disclosed herein a signal pattern comprising signals aberrant from wild type signals and/or the ratio of signal integrals of a reference signal and a signal to be analysed are built. The signals employed for building the pattern are chosen to allow for a reproducible correlation of the pattern to the particular somatic mutation phenotype.

Microsatellites are the most common class of inter- or intragenic repetitive sequences occurring in eucaryotic genomes. They comprise a minimal repetitive unit of one to six bases in length. These sequences are highly polymorphic in human populations and may be used for carrying out linkage analysis (Weber, May et al., 1989; Dib et al. 1996). Although these polymorphisms are highly polymorphic compared to other genomic regions they seem to be stable in single individuals. In some cancers such as HNPCC it could be shown, that microsatellites become unstable (Thibodeau et al. 1993; Aaltonen et al,. 1993).

It is well known, that aberrantly incorporated nucleotides, which have been incorporated e.g. by slippeage of the DNA polymerases in the course of the replication process are usually removed by DNA mismatch repair (Parker and Marinus, 1992; Johnson et al., 1996; Palombo et al. 1996).

In cases where the respective enzymes engaged in DNA mismatch repair are defective, errors as described above accumulate. The defects in DNA mismatch repair genes are of various origins and may thus be hardly detected. Nonetheless these defects may be present in promoter regions leading to insufficient or absent expression of the mismatch repair genes.

The detection of instability of microsatellite regions thus provides an efficient screening approach for indirect detection of mutations in DNA mismatch repair genes. The phenotype of microsatellite instability provides a valuable indicator for cells harbouring defects in DNA mismatch repair mechanisms and provides thus a marker for cells of elevated risk to develop cancerous properties.

The analytical method for detection of microsatellite instability used in the art vary widely, leading to different and in part incompatible results of the analysis. This discrepancy between the methods may on the one hand be dependent on different microenvironments in different tissues and on the other hand may be caused by the different analytical methods themselves. Due to this fact the results generated by different investigators may not be directly compared to each other. As a result of this unsatisfying situation the "International Workshop on Microsatellite Instability and RER Phenotypes in Cancer Detection and Familial Predisposition"established and recommended guidelines for diagnosis based on microsatellite instability.

The common methods for the analysis of somatic mutations in repetitive sequences are either based on PCR techniques directly addressing variations in the genome or are based on the inactivation of particular markergene sequences. The second method however is not suited for use in medical applications, for the reporter constructs have to be introduced into the cells which have to be analysed. Thus this method is predominantly restricted to use in yeast systems. In clinical diagnostics PCR based approaches are dominating. The amplified microsatellite regions generate fragments of different lengths and amounts. Similar fragments may also be generated due to the polymerases used in the PCR technique. Thus PCR products of different samples are hardly comparable.

The methods used in the art for analysis of somatic mutations in microsatellite regions show a series of disadvantages and make the results of the investigations prone to artefacts. A global objective analysis of the results of a larger number of research laboratories is hindered by the fact, that the results are not always compatible and comparable to each other.

Thus a method which allows for standardization of the results produced by analysis of somatic mutations in microsatellite regions for detection of microsatellite instability phenotype would be desirable.

It could be expected, that mass spectrometric methods for determination of molecular weights of DNA fragments may lead to faster more reliable results of enhanced fidelity compared to the methods based on the detection of electrophoretic or chromatographic mobility. Beside a better mass resolution mass spectrometry in contrast to mobility based techniques is not susceptible to secondary or tertiary structures of the analyte molecule. It is well known that such interferences could lead to wrong interpretations of the nature of analyte molecules.

For the mass spectrometric detection ionisation of DNA fragments may be done by the common methods such as electron spray ionization (ESI) or matrix assisted laser desorption and ionization (MALDI). Mass spectrometers such as high frequency quadrupole ion traps, ion cyclotron resonance spectrometers or time of flight mass spectrometers may be applied for theses purposes. Especially useful may be a combination of MALDI and a time of flight mass spectrometer (MALDI-TOF). The present rapid progression in development of the MALDI technology allows for a high degree of automatization of ionisation of samples and such reduces the time necessary for analysis of single samples.

The mobility of ions in a time of flight mass spectrometer is about 107 times that given in electrophoretic gels. Given that 10 to 100 spectra will be needed for generation of suitably reliable signals the mass spectrometry still remains faster than other methods. The mass spectrometry allows to determine analyte molecules up to 15.000 molecular mass units corresponding to about 40 single strand bases in amounts of few femtomols within analysis times of few seconds.

In contrast to gel electrophoresis mass spectrometry yet is not suited for analysis of larger DNA fragments. Currently polynucleotides greater than 60 bases in length can hardly be subject to mass spectrometric mass determination. Yet in the range of small DNA fragment molecules the accuracy achievable by mass spectrometry by far exceeds that given by gel electrophoresis. For fragments of 20 bases and less a determination of the molecular masses with a precision of up to one tenth of a molecular mass unit is possible. Fragments of up to 30 bases render a precision of one half mass unit and fragments of up to 40 bases render fidelities of about five mass units. Even the smallest possible difference in mass resulting from a single base exchange (9 mass units) is still reliably detectable by mass spectrometry. Thus the accuracy and precision of the mass spectrometric analysis by far exceeds the accuracy and precision of electrophoretic or chromatographic methods. This is especially true for small fragment sizes in the analyte samples. The use of mass spectrometry for the analysis of short microsatellites has several advantages compared to electrophoretic or chromatographic analysis of commonly used long microsatellites.

The methods used in the art for the detection of microsatellite instabilities in tissues are based on the detection of dinucleotide repeat tracts. These tracts are of sizes, which are no longer accessible to reliable detection of mutations by mass spectrometry. Thus in order to employ mass spectrometric processes for determination of microsatellite instabilities new substitutes for the commonly used markers have to be established, that ensure reproducible results of suitable accuracy and precision. Commonly used markers have properties well suited for electrophoretic or chromatographic analysis such that their size and their possible differences in size can be analysed by these techniques. However small repetitive units such as mono nucleotide repeats are not well suited for analysis by these techniques because their size is usually in the range of 8-50 bases and varies in unstable samples by only one or two bases (n-1, n-2 and/or n+1, n+2). Only high resolution sequencing gels are able to analyse those differences. Therefore the present invention also comprises substitutes for commonly used microsatellite markers such that they are suitable for mass spectrometric analysis without loosing the information provided by markers commonly used in the field.

Another problem with the use of mass spectrometric methods for the detection of microsatellite instabilities in tissues, is, that the mutations in question in the microsatellite regions are somatic mutations. A key feature of somatic mutations is the heterogeneity of tissues with respect to the named mutations. Thus samples used for the detection of the somatic mutations indicative for the microsatellite instability phenotype comprise sequences with mutations within the microsatellite regions as well as sequences without mutations. A detection of the mutations has to be carried out in a mixture containing different ratios of non-mutated and mutated sequences. The situation furthermore is complicated by the fact, that amplification reactions of the repeat tracts inherently produce amplification products which result from DNA polymerase slippeage (stutter peaks), which are shorter or longer than the template sequence. Thus even amplification of wild type samples of microsatellites generates these artefact peaks, which mimic -1 or -n mutations in the microsatellite regions. A problem like this is solved by electrophoretic means, where an additional band of a particular intensity is rendered by the mutation. The intensity is different in the wild type samples. Thus comparison of gels produced from wild type samples with those of the samples in question allows for detection of somatic mutations in microsatellite regions.

In mass spectra in contrast the determination of somatic mutations is a more sophisticated task. The process of mass spectrometric analysis renders different spectra for each ionisation event and thus for each single spectrum. Due to this fact it is not possible to determine whether a somatic mutation is present by comparing the peak intensities present in different spectra.

Thus it would be desirable to provide a method, that enables for mass spectrometric analysis of somatic mutations, such as mutations in microsatellite regions. A method that fulfils this need in the art is provided by the present invention.

The present invention is based on the inventors' findings, that spectra of wild-type microsatellites show a specific signal pattern different from mutated microsatellites. This enables the clear detection of mutated molecules present in a sample. The setting of a specific pattern for wild-type samples by means of signal intensities and signal positions for diagnostic purposes allows to detect somatic mutations, which are present together with non-mutated sequences in a single sample by any pattern aberrant from the wild type pattern. According to the present invention the ratio of the integral over the range of the peak rendered by the wild type length microsatellite to the integral referring to the mutated variation of the microsatellite may be used as an indicator for the presence or absence of a microsatellite instability phenotype in a tumor sample.

The inventors found that the signal patterns of the wild type and the mutated signals in samples are reproducibly correlated with microsatellite stability or microsatellite instability phenotypes, for the sequences giving rise to the respective peaks are nearly identical and so the ionisation event as well as the subsequent analysis event in a TOF mass spectrometer are inherently very similar within a single experiment. The differences arising from different experimental procedures pertain to the signals in the same manner, such that the ratio of these signals and/or their position is comparable irrespective of the inter-experimental differences characteristic for mass spectrometric methods.

To overcome the problem presented by the restricted lengths measurable by mass spectrometry the inventors have found, that especially mononucleotide repeats, which commonly are shorter than e.g. dinucleotide or trinucleotide repeat tracts are in a range, that is well suited for mass spectrometric analysis.

The present invention relates to a method for the detection of somatic mutations in microsatellite regions. The method is based on the determination of signal patterns comprising the presence or absence of signals in given mass to charge range and/or the ratio of a reference signal to analyte signals characteristic for the presence of a somatic mutation.

In a second aspect the invention provides a method for the classification of tumors according to the microsatellite instability phenotype using a panel of mononucleotide repeat tracts as markers.

In a third aspect the invention provides a test kit for performing the detection reactions described herein and furthermore a kit for performing the classification according to the present invention.

Somatic mutation as used in the context of the present invention is any mutation, which has not been inherited by an individual, but which has been acquired during the individuals' lifetime. A characteristic of the somatic mutations as used herein is, that they are restricted to particular tissues or even parts of tissues or cells within a tissue and are not present in the whole organism harbouring the tissues or cells. Examples of somatic mutations include mutations produced by mismatch incorporations of nucleotides during replication of the genomic DNA in the course of the cell division cycle of proliferating cells. In one embodiment of the present invention such somatic mutations may be mutations in coding and/or non-coding microsatellite regions. In one preferred embodiment of the present invention the microsatellite regions are mononucleotide repeat tracts. Mutation as used in the context of the present invention may comprise insertions or deletions of one or several nucleotides (or nucleotide repeats) within nucleotide sequences.

Microsatellite regions as used in the context of the present invention are stretches of repetitive elements in polynucleotides. The microsatellites may for example comprise several repeats of one single nucleotide or several repeats of two, three up to six nucleotides. In microsatellites the number of repeats of the repetitive unit ranges from 3 to 15 or even more repeats of said unit. In one preferred embodiment of the invention the microsatellites comprise mononucleotide tracts of lengths up to 15 repeats.

The method according to the present invention may be used for the detection of somatic mutations. Disorders, to which the method according to the present invention may be applied comprise any disorders associated with somatic mutations within coding and/or noncoding regions of genes. These disorders comprise for example proliferative and degenerative diseases, such as neurodegenerative diseases, vascular disorders, disorders caused by stress, such as oxidative stress, chemically induced stress, radiation induced stress and cancers including all sporadic cancers as well as HNPCC associated cancers. Cancers as used herein may comprise e.g. colorectal cancer, small cell lung cancer, liver cancer (primary and secondary), renal cancer, melanoma, cancer of the brain, head and neck cancer, gastrointestinal cancers, leukemia, lymphomas. prostate cancer, breast cancer, ovary cancer, endometrial cancer, lung cancer, bladder cancer etc..

The method according to the present invention may be applied to any organisms prone to get affected by degenerative disorders. The organisms are for example mammalian animals and especially animals of agricultural value such as pigs, cows, sheep, etc., companion animals, such as cats, dogs, horses etc., animals employed in research purposes such as mice, rats, rabbits, hamsters etc. or humans.

A sample according to the method of the present invention may comprise any sample comprising nucleic acids from tissues affected by a disorder or suspected of being affected by a disorder associated with somatic mutations as described above. Thus samples may comprise samples of clinical relevance, such as e.g. body fluids, such as blood, plasma, serum, secretions, smears, urine, semen, bile, liquor, etc. Furthermore samples may comprise stool, biopsies, cell- and tissue-samples. Biopsies as used in the context of the present invention may comprise e.g. resection samples of tumors, tissue samples prepared by endoscopic means or needle biopsies of organs.

Before application of the method according to the present invention to the samples appropriate methods for extraction of the DNA out of the samples have to be applied. The methods useful are known to those of skill in the art.

Nucleic acids of interest for the method of the present invention comprise genomic sequences such as intergenic sequences, genes, microsatellites etc.. In one embodiment of the present invention the. sequences are sequences, that are known to be somatically mutated in association with degenerative disorders.

For use in the method according to the present invention the nucleic acids of the samples have to be prepared in a way that allows for an analysis of the nucleic acids by means of mass spectrometry.

In one embodiment of the invention nucleic acids are prepared from the samples by the methods known to those of ordinary skill in the art. The nucleic acids for use in the method according to the present invention may for example include single-stranded (sense or antisense) or double-stranded molecules, and may be DNA (genomic, cDNA or synthetic) or RNA. RNA molecules comprise as well hnRNA (containing introns) as mRNA (not containing introns).

Subsequently the purified nucleic acids may be subjected to amplification reactions. Suitable amplification reactions are known to those of ordinary skill in the art and may comprise DNA based amplification as well as RNA based amplification. Amplification reactions according to the present invention may comprise PCR, LCR, NASBA, etc.. The amplification reaction may be performed using one or more specific primers. In one embodiment of the invention an amplification reaction comprises the amplification of a single nucleic acid. In another embodiment of the invention the amplification is carried out as a multiplex amplification reaction simultaneously amplifying a set of several nucleic acids.

In one embodiment of the present invention the amplified nucleic acids may be used for a subsequent primer extension reaction, with or without prior purification which gives rise to nucleic acid fragments of 10 to about 50 bp length.

In one embodiment of the present invention the amplified nucleic acids may but not need to be subjected to a purification process prior to the direct mass spectrometric analysis to further enhanced signal to noise ratios.

The mass spectrometric analysis as method according to the present invention may generally be performed using any type of mass spectrometer suitable for the particular purpose. Any kind of mass analyser can be used unless it is capable of analysing macromolecules without further fragmentation. The ionisation method usable for the present invention in one preferred embodiment is soft by means of preventing the analyte molecule from extended decomposition during the ionisation process.

Ionisation methods suitable for soft ionisation may comprise for example the matrix assisted laser desorption and ionisation (MALDI) and electrospray ionisation (ESI). MALDI is usually used in a high vacuum environment whereas ESI is an atmospheric pressure ionisation method. Both methods can be coupled to any kind of mass analyser suitable to the present invention.

For example the mass spectrometric analysis may comprise an analysis of the mass per charge of the analyte by means of storing the ions in an electric field and releasing stored ions sequentially according to their mass per charge ratio in an ion trap mass analyser. In one embodiment of the invention the mass analysis is characterized in that it utilizes the linear dependency between the time of flight and the mass per charge ratio in a time of flight (TOF) mass analyser.

In one embodiment of the invention the ionisation method is characterized in that it predominantly generates single charged molecules, which directly exhibit the molecular mass of a given species in a counts-mass/charge. In another embodiment a method is used, that generates spectra with multiple charged molecules leading to one or several signals for each analyte molecule.

A mass spectrometric method for use in the present invention may for example comprise the cocrystalization of the sample together with a matrix of small organic compounds (MALDI). The small organic compounds may comprise picolinic acid and/or its derivatives if nucleic acids are being analysed.

The mass spectrometric analysis may furthermore comprise the evaporation of the sample under atmospheric pressure by spraying the analyte under a constant flow through a capillary under high voltage directly into the mass analyser (ESI).

In one embodiment of the invention the ion source may be coupled directly to an online purification system which allows a further reduction of analysis time.

In one embodiment of the present invention the inventive method is used for the detection of a microsatellite instability phenotype. Determination of a microsatellite instability phenotype according to the present invention may comprise the detection of microsatellite instability in a mononucleotide repeat tract of nucleic acids of a sample. Suitable mononucleotide tracts may comprise e.g. the repeat tracts of the following genes: GARS/AIRS-GART (**Locus** 21q22.1 **Locus ID:** 2618 **UniGene:**Hs.82285), AC1 (**Locus:** 4p16 **Locus ID:** 10141 **UniGene:**Hs.177972), TGFBRII (**Locus:** 3p22 **Locus ID:** 7048 **UniGene:** Hs.82028), MSH3 **(Locus:** 5q11-q12 **Locus ID:** 4437 **UniGene:** Hs.42674), MSH6 (**Locus:** 2p16 **Locus ID:** 2956 **UniGene:** Hs.3248), KIT (**Locus:** 4q12 **Locus ID:** 3815), MSH2 (**Locus:** 2p22-p21 **Locus ID:** 4436)

Another aspect of the present invention is a kit for performing the methods disclosed herein. A kit according to the present invention comprises at least an agent suitable for detecting the molecules disclosed herein. Furthermore a kit according to present invention may comprise:
a) one or more specific nucleic acid probes for use in a nucleic acid amplification reaction
b) reagents used in nucleic acid amplification reaction comprising one or more polymerase enzymes, substrates for the amplification reaction, buffers and/or activators
c) compounds for use in the conditioning of samples or nucleic acids comprising (magnetic) beads, devices for chromatography, ready to use tubes and/or filters and/or membranes.
d) reagents and buffers suitable for the preparation of genomic DNA from given sample tissues and/or stool and/or blood and/or sputum.
e) additional software for the automated sample conditioning and/or automated analysis of raw data generated by a mass spectrometer.
f) packings of said kit according to the present invention suitable for use in automated pipetting systems

The present invention provides a method for the detection of somatic mutations by means of mass spectrometry. The method thus enables for an accurate, reproducible and rapid analysis of samples for the presence or absence of somatic mutations. The invention furthermore provides a method for the detection of a microsatellite instability phenotype in samples. The method for the detection of the microsatellite instability phenotype comprises the detection of somatic mutations in microsatellite regions of one or more genes selected from a group consisting of GARS/AIRS-GART **(Locus** 21q22.1 **Locus ID:** 2618 **UniGene:**Hs.82285), AC1 **(Locus:** 4p16 **Locus ID:** 10141 **UniGene:**Hs.177972), TGFBRII **(Locus:** 3p22 **Locus ID:** 7048 **UniGene:** Hs.82028), MSH3 **(Locus:** 5q11-q12 **Locus ID:** 4437 **UniGene:** Hs.42674), MSH6 (Locus: 2p16 **Locus ID:** 2956 **UniGene:** Hs.3248,) , KIT **(Locus:** 4q12 **Locus ID:** 3815), MSH2 **(Locus:** 2p22-p21 **Locus ID:** 4436)
and wherein the disorder is diagnosed to be of a microsatellite instability phenotype if at least 1 of the analysed genes show somatic mutations. Another aspect of the present invention is a test kit for performing the methods disclosed herein.

### Brief description of the Drawings:

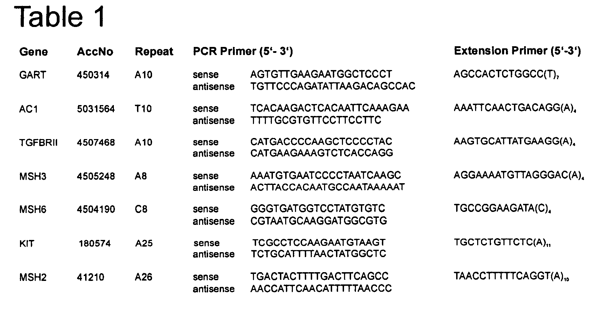

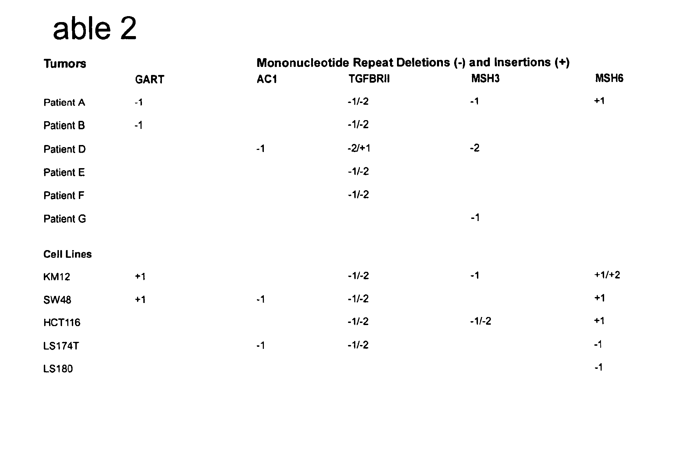

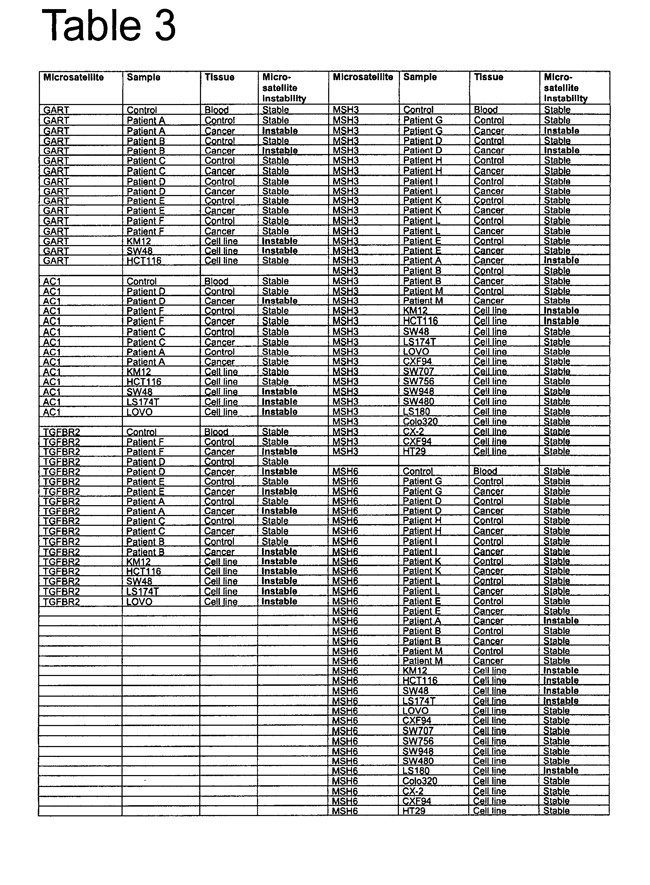

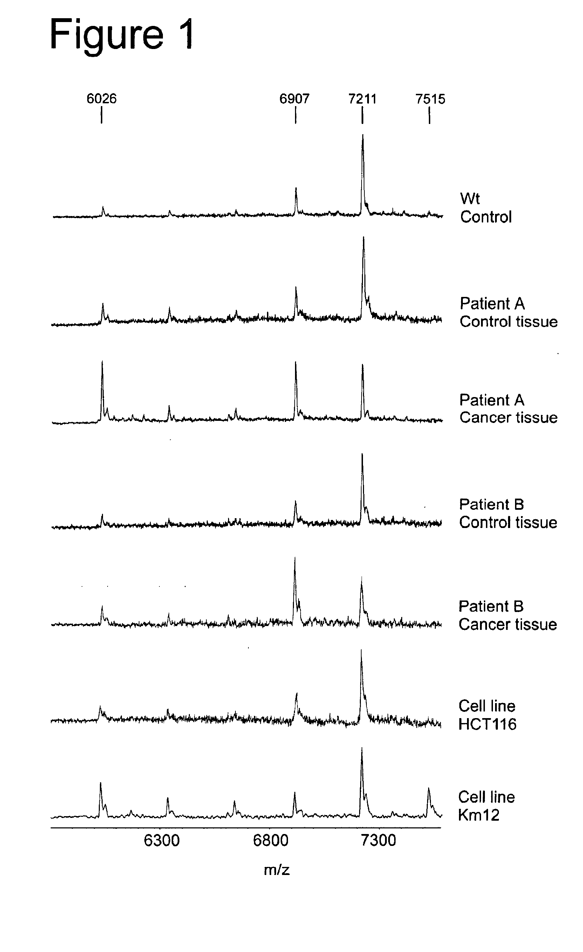

- Table 1:: **Sequences of the primers used for PCR or extension reactions of the investigated microsatellites**
- Table 2:: **Detected mononucleotide repeat deletions and insertions in the GART, AC1, TGFBRII, MSH3 and MSH6 genes of tumors in different primary tumors and cell lines**
- Table 3:: **Results from MALDI-TOF-MS analysis of different microsatellites** due to the comparison of the complex signal pattern of control and tissue/cell line spectra
- Figure 1:: **MALDI-TOF-MS analysis of microsatellite instabilities in the GART gene.** MALDI-TOF-MS spectra of control DNA, DNA of patient A (control tissue and cancer tissue) and patient B (control tissue and cancer tissue) as well as DNA of cell line KM12. The remaining unextended primer (6026Da) were used for internal mass calibration. The molecular mass of 7211Da represented the normal allele, while the molecular masses of 6907Da and 7215Da indicated the deletion and insertion of one A in the microsatellite, respectively. Whereas the patients A and B control tissues show the same signal pattern compared to the stable microsatellite control spectrum a significant difference in the signal pattern can be observed in spectra derived from cancer tissue and the cell line KM12. Whereas the tumors show a reduction in length (-1 base) indicated by a change in signal intensities of the wt signal (7211) and the n-1 signal (6907) the cell line shows an increase in length by 1 base indicated by a new arising signal at 7515.

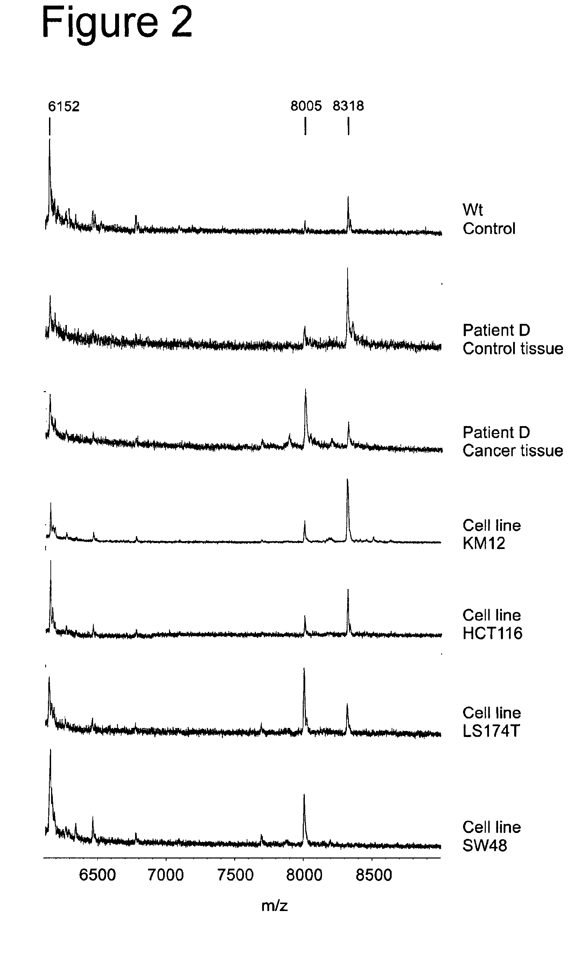

- Figure 2:: **MALDI-TOF-MS analysis of microsatellite instabilities in the AC1 gene.** MALDI-TOF-MS spectra of control DNA, DNA of patient D (control tissue and cancer tissue) and DNA of cell lines KM12, HCT116, and SW48. The remaining unextended primers (6152 Da) were used for internal mass calibration. The molecular mass of 8318 Da represented the normal allele, while the molecular mass of 8005 Da indicated the deletion of one T in the microsatellite. Spectra derived from patient D cancer tissue and cell lines LS174T show different signal patterns by reverting their signal intensity compared to the wt control which indicates MSI. Cell line SW48 does not show microsatellite instability phenotype; this may be concluded from the fact that the AC1 is shortened by 1 base in all cells and is therefore not a somatic mutation but a model for germ line mutation.

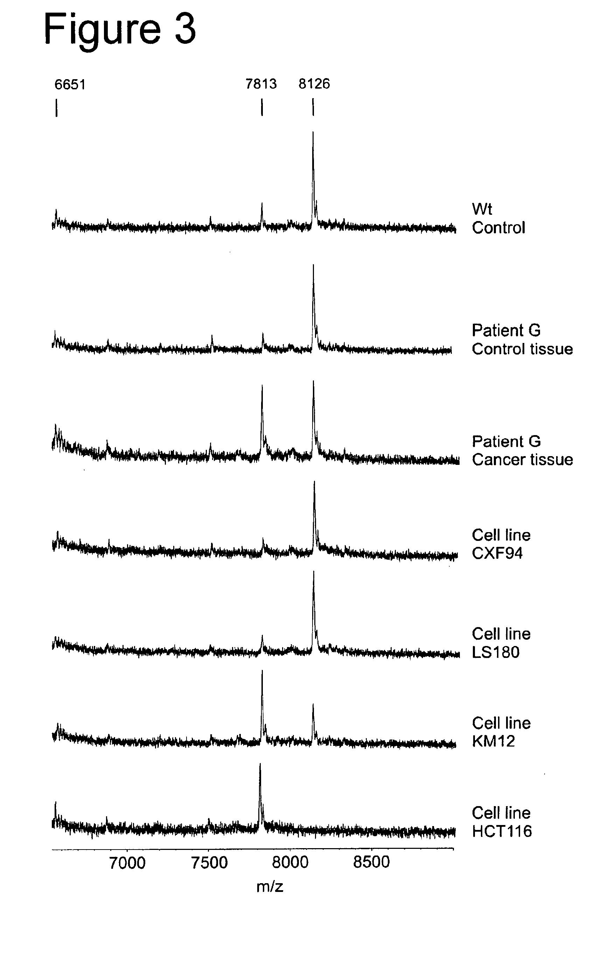

- Figure 3:: **MALDI-TOF-MS analysis of microsatellite instabilities in the MSH3 gene.** MALDI-TOF-MS spectra of control DNA, DNA of patient G (control tissue and cancer tissue) and DNA of cell lines KM12 and HCT116. The remaining unextended primers (6651 Da) were used for internal mass calibration. The molecular mass of 8126 Da represented the normal allele, while the molecular mass of 7813 Da indicated the deletion of one A in the microsatellite. Signals between these masses indicate the slippeage of the DNA polymerase and contribute to the internal background noise. The signals at 7813 are significantly enlarged compared to the wt control spectra in the MSH3 microsatellite from patient G and cell line KM12 indicating microsatellite instability for MSH3 in these samples.

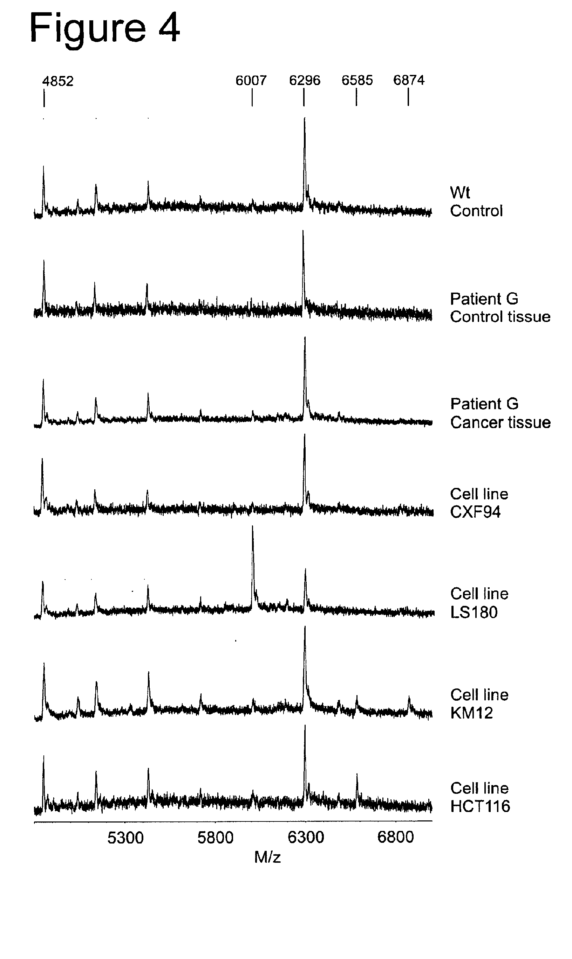

- Figure 4:: **MALDI-TOF-MS analysis of microsatellite instabilities in the MSH6 gene.** MALDI-TOF-MS spectra of control DNA, DNA of patient G (control tissue and cancer tissue) and DNA of cell lines LS180, KM12 and HCT116. The remaining unextended primers (4852 Da) were used for internal mass calibration. The molecular mass of 6296 Da represented the normal allele, while the molecular masses of 6007 Da, 6585 and 6874 Da indicated the deletion of one A, insertion of one or two A in the microsatellite, respectively. Only cell line CXF94 and samples derived from patient G do not show instability. The investigated cell lines LS180 (-1 deletion) KM12 (insertion of one and two bases) and cell line HCT116 (insertion of one base) show instability indicated by the addition of new signals at 6007 (cell line LS180), 6585 (HCT116) and 6874 (KM12).

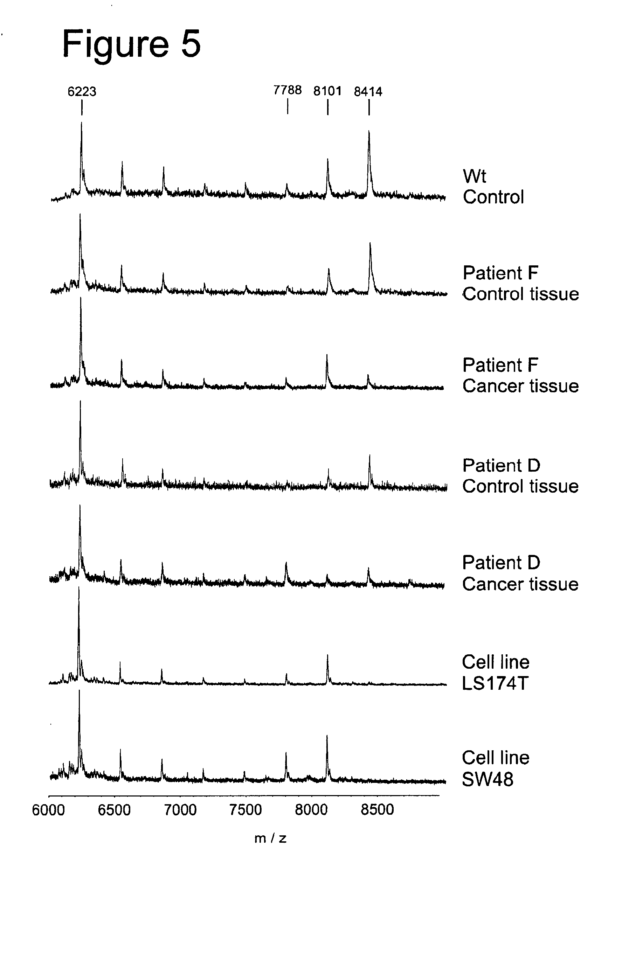

- Figure 5:: **MALDI-TOF-MS analysis of microsatellite instabilities in the TGFBR2 gene.** MALDI-TOF-MS spectra of control DNA, DNA of patient F (control tissue and cancer tissue) and patient D (control tissue and cancer tissue). The remaining unextended primers (6223 Da) were used for internal mass calibration. The molecular mass of 8414 Da represented the normal allele, while the molecular mass of 8101 Da indicated the deletion of one A and a molecular mass of 7788 Da indicated the deletion of two A in the microsatellite. Patient F's as well as patient D's cancer tissues show MSI indicated by a change in signal intensities of the signals at 8101, 7788 and 8414 compared to the wild type spectrum. The investigated cell lines are stable for TGFBR2.

The following examples are given for illustration and are not intended to limit the scope of the present invention.

### Example1: Diagnosis of microsatellite instability by means of MALDI-TOF-MS

Microsatellite instability has been diagnosed from the MALDI-TOF-MS spectra if differences in the samples complex signal patterns compared to the control had been observed. Lack of signals as well as addition of signals or differences in signal shape and/or mixtures of all of these markers have been indicators for instability.

All results have been confirmed by conventional sequencing.

The experiments have been performed as follows:

Samples were obtained from HNPCC patients or from cell lines. Genomic DNA was extracted from microdissected patient tissue or cell lines by using the RapidPrepTM Macro Genomic DNA Isolation Kit (Pharmacia, Germany) according to the manufacturer's protocol.

### Amplification of microsatellites from genomic DNA

The microsatellites as indicated in the schemes have been amplified by using the appropriate primers. Each PCR reaction (10 µL) contained 12 pmol of both forward and reverse primers, 50ng of genomic DNA template, 1 U PAN Taq Polymerase in supplied NH4-buffer supplemented by 1.5 mM MgCl2, and 2 nmol of each dNTP. Thermal cycling conditions were 96°C for 2 min, 35 cycles at 96°C for 60 s, 60°C for 30 s, and finally 72°C for 3 min.

### Allele specific extension of microsatellite PCR product

The allele specific extension reaction after addition of extension mix (10 µL), containing 12 pmol extension primer (for primer sequences please see the scheme for each microsatellite), 1 to 2 U

Thermosequenase, Thermosequenase reaction buffer (Pharmacia, Germany), 2 nmol assay specific dNTPs and ddNTPs (for appropriate NTPmix please see the scheme for each microsatellite).

Extension reactions were performed at 96°C for 2 min, and 40 cycles at 94°C for 30 s, 55°C, respectively, for 30 s and finally 72°C for 3 min.

### Sample purification

Both, PCR and primer extension products were purified using magnetic beads (GenoPure) as described by the supplier (Bruker Daltonik GmbH, Bremen, Germany).

### MALDI-TOF mass spectrometry

Aliquots of the purified samples were spotted onto matrix crystals of 3-hydroxypicolinic acid on a stainless steel target and air dried. Mass determinations were performed on a Biflex™ III MALDI-TOF mass spectrometer (Bruker Daltonik GmbH, Bremen, Germany) equipped with a nitrogen laser (337 nm) and delayed extraction. Laser-desorbed positive ions were analysed following acceleration by 20 kV in the linear mode. External calibration was performed using a standard oligo-nucleotide mixture.

### Conclusion

The present example clearly shows the applicability of the present invention in the reliable detection of MSI in tissue samples from patients as well as in cell lines. Different complex signal patterns enable the reliable detection of a variety of instabilities such as insertions and deletions of several bases without ambiguities. All experimental data could be confirmed by independent methods used in the art. The clear differences in signal patterns of instable microsatellites compared to stable microsatellites also enable a fully automated analysis of MSI in clinical relevant tissue samples from patients.

## Claims

1. A method for the detection of somatic mutations in samples comprising
a. preparing nucleic acids from said sample, such that they may be analysed by mass spectrometry
b. analysing the nucleic acids by means of mass spectrometry
c. determining the presence or absence of at least one complex pattern comprising a new signal and/or a given ratio of at least two signal integrals within the mass spectra of said nucleic acid preparations characteristic for the presence or absence of a somatic mutation,
i. wherein the signal integrals for getting a ratio are chosen to be the integral over the area of the signal preferentially of an unmutated nucleic acid sequence and the integral over the area of a peak of the correlated mutated nucleic acid sequence and
ii. wherein the ratio is characteristic for the presence of a somatic mutation, when the integral over the peak area of mutated sequence is equal to or greater than the integral over the peak area of the unmutated sequence and/or a signal is observed which is aberrant from any wild type spectrum; and
d. assessing the presence or absence of a somatic mutation within the sample from said ratio.

2. The method according to claim 1, wherein the nucleic acids are intergenic sequences, genes, or microsatellites.

3. The method according to claim 2, wherein the sequences are sequences known to be somatically mutated in proliferative or degenerative disorders.

4. The method according to claims 2 or 3, wherein the nucleic acids analysed by mass spectrometry are nucleic acid fragments

5. The method according to claim 4, wherein the fragments are of lengths of no more than 50 bp.

6. The methods of any of the preceding claims, wherein the preparation of the nucleic acids comprises an amplification step.

7. The method according to claim 6, wherein the amplification step is PCR, LCR or NASBA.

8. The method according to any one of the preceding claims, wherein the nucleic acid preparation comprises the simultaneous amplification of multiple nucleic acid sequences in the process of a multiplex amplification reaction such as multiplex PCR.

9. The method according to any one of the claims 6 to 8, wherein the amplification product is directly subjected to mass spectrometric analysis.

10. The method according to any one of the preceding claims, wherein the mass spectrometric analysis is carried out by MALDI TOF MS or ESI MS.

11. The method according to claim 10,.wherein the mass spectrometric analysis furthermore employs a reflector for precise determination of masses.

12. The method according to any one of the preceding claims, wherein the analysis of the spectra comprising the building of said ratios is carried out automatically employing a suitable computer software.

13. A method according to any one of the preceding claims, wherein the steps are performed in the course of a fully automated process.
A method of any one of the preceding claims, which is used for the detection of the presence or absence of the microsatellite instability phenotype of disorders, wherein at least 1 nucleic acid sequence chosen from a group consisting of: GARS/AIRS-GART **(Locus** 21q22.1 **Locus ID:** 2618 **UniGene:**Hs.82285), AC1 **(Locus:** 4p16 **Locus ID:** 10141 **UniGene:**Hs.177972), TGFBRII **(Locus:** 3p22 **Locus ID:** 7048 **UniGene:** Hs.82028), MSH3 (**Locus:** 5q11-q12 **Locus ID:** 4437 **UniGene:** Hs.42674), MSH6 (Locus: 2p16 **Locus ID:** 2956 **UniGene:** Hs.3248), KIT **(Locus:** 4q12 **Locus ID:** 3815), MSH2 (**Locus:** 2p22-p21 **Locus ID:** 4436)

14. is analysed and wherein the disorder is diagnosed to be of a microsatellite instability phenotype if at least 1 of the analysed genes show somatic mutations.

15. The according to claim 14, wherein additionally one or more further microsatellite regions are analysed.

16. The method according to claim 14 or 15, wherein the analysis of one or more nucleic acids is carried out simultaneously.

17. A kit for performing a method according to any one of the preceding claims being a research kit or a diagnostic test kit.

18. The kit of claim 17, comprising at least one of the following
a. one or more specific nucleic acid probes for use in a nucleic acid amplification reaction
b. reagents used in nucleic acid amplification reaction comprising one or more polymerase enzymes, substrates for the amplification reaction, buffers and/or activators
c. compounds for use in the preparation and/or purification of samples or nucleic acids comprising (magnetic) beads, devices for chromatography, ready to use tubes and/or filters and or membranes.

19. The kit according to any one of the claims 17-18, wherein all components are prepared for use in an automated process employing pipetting robots.
